# EUROPEAN PATENT SPECIFICATION

(11) **EP 3 044 306 B1**
(45) Date of publication and mention of the grant of the patent: **11.03.2020**
(21) Application number: 14796257.5
(22) Date of filing: 09.09.2014
(51) Int. Cl.: C12M 1/00, C12M 3/00, C12M 1/12

(54) **SUPPORT FOR CELL CULTURES**
TRÄGER FÜR ZELLKULTUREN
SUPPORT POUR CULTURES CELLULAIRES

(30) Priority: 10.09.2013 IT MI20131494
(43) Date of publication of application: 20.07.2016
(73) Proprietor: Universita' Degli Studi di Pisa, 5614 Pisa (IT)
(72) Inventor: SBRANA, Tommaso, I-55054 Massarosa (Lucca) (IT); MATTEI, Giorgio, I-53036 Poggibonsi (Siena) (IT); AHLUWALIA, Arti, I-54035 Fosdinovo (Massa Carrara) (IT); GIUSTI, Serena, I-57034 Campo Nell'elba (Livorno) (IT)
(74) Representative: Lunati & Mazzoni S.r.L.
(86) International application number: PCT/IB2014/064330
(87) International publication number: WO 2015/044813

(56) References cited:
- WO-A1-2012/162855
- US-A- 5 665 599
- US-A1- 2011 136 218

## Description

The present invention relates to an innovative support for cell constructs and/or cell cultures of the type as recited in Claim 1.

As is known, preparing and analyzing in vitro cell cultures is particularly demanding owing to the need to realistically simulate the natural environment and, thus, to simulate the biochemical, mechanical and dynamic interactions to which the cells are subjected.

The standard method for culturing and analysing cell constructs involves first arranging the cell construct on a slide in a culture support in which the cells adhere and differentiate appropriately in order to achieve the colonisation of the construct. Next, it is placed in a bioreactor where, under the appropriate stimuli, the physiological environment is mimicked. Monitoring with a microscope is performed during an initial cell growth phase and following dynamic stimulation, when samples are taken and the construct is thus removed from the bioreactor. Said bioreactors therefore comprise two bodies that can be reciprocally coupled so as to define an airtight chamber and a supporting surface for the cell construct; and stimulating means suitable to stimulate the cell construct such as, for example, ducts defining a flow of fluid that enters and/or leaves the chamber or a stimulator suitable to vary the pressure of the fluid acting on the cell construct. US 5,665,599 discloses a chamber for cultivating cells.

The prior art solution mentioned above has several significant drawbacks.

A first important drawback lies in the fact that the process of culturing and analysis is extremely complex.

Said drawback is due to the need to handle the cell construct and, in particular, to remove it first from the support for cell cultures and, subsequently, from the bioreactor and to place it under a microscope or in other test equipment without contaminating or altering the cell construct.

Another drawback consists of the fact that, with the methods known in the prior art, it is impossible to analyse the cell construct during its stimulation and thus in real-time.

A further drawback is given by the complexity of monitoring the construct during the culturing process, that is to say while it is in the support.

In this situation the technical purpose of the present invention is to devise an innovative support for cell cultures able to substantially overcome the drawbacks mentioned above.

Within the scope of said technical purpose an important aim of the invention is to provide a support for cultures that makes it possible to simplify and speed up the analysis of a cell construct and, in particular, which permits real-time analysis using optical or other means.

In particular, an important purpose of the invention is to obtain a support for cell cultures which makes it possible to analyse a cell construct while minimising the risk of contamination or alteration.

A further purpose of the invention is to devise a support which allows the construct to be monitored simply and quickly while in the support.

The technical purpose and specified aims are achieved with an innovative support for cell cultures as claimed in the appended Claim 1.

Preferred embodiments are described in the dependent claims.

The characteristics and advantages of the invention are clearly evident from the following detailed description of a preferred embodiment thereof, with reference to the accompanying drawings, in which:
**Fig. 1** shows a support for cell cultures according to the invention;
**Fig. 2** is an exploded view of the support for cultures shown in Fig. 1;
**Fig. 3** illustrates a further example of a support for cell cultures;
**Fig. 4** represents an exploded view of the support for cultures shown in Fig. 3;
**Fig. 5** is a further example of a support for cell cultures;
**Fig. 6** shows an exploded view of the support for cultures shown in Fig. 5;
**Fig. 7** is a further example of a support for cell cultures;
**Fig. 8** shows an exploded view of the support for cultures shown in Fig. 7;
**Fig. 9a** illustrates an exploded view of a detail of the support in Fig. 8; and
**Fig. 9b** shows another view of the part shown in Fig. 9a.

With reference to said drawings, reference numeral 1 globally denotes the innovative support for cell cultures according to the invention.

The support 1, developed within the scope of the Re-Liver project funded by the EU Seventh Framework Programme (FP7/ 2007-2013) under grant agreement No. 304961, comprises a culture chamber **10,** which is substantially circular and airtight, defining a preferred axis of extension **11,** substantially parallel to the gravitational gradient, a supporting surface **12** for the cell construct and a distal surface **13** with respect to said supporting surface 12; a first body **20** defining a first portion of the culture chamber 10 and a second body **30** defining a second portion of the culture chamber 10 complementary to said first portion and suitable to be coupled with the first body 20 so as to define the culture chamber 10.

The second body 30 is provided with a recess **31** defining the distal surface 13 and substantially the second portion of the chamber 10; at least one delivery duct **32** suitable to permit a fluid to enter the culture chamber 10; and at least one discharge duct **33** suitable to permit the fluid to leave the chamber 10 and having an inside diameter that is substantially larger than and, in particular, twice that of the inside diameter of the delivery duct 32.

The distal surface 13 of the recess 31 is appropriately perpendicular in relation to the preferred axis of extension 11.

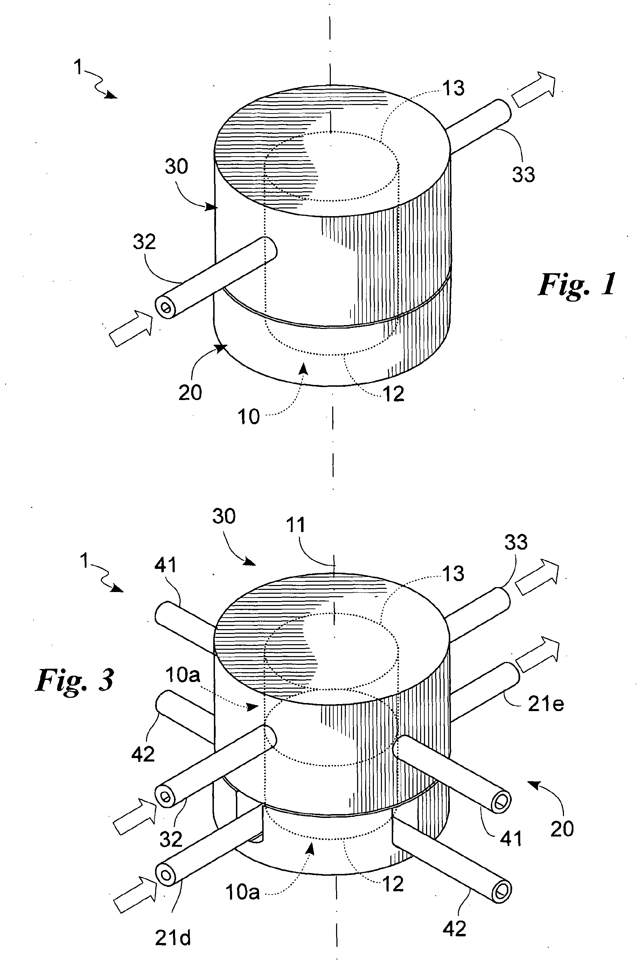

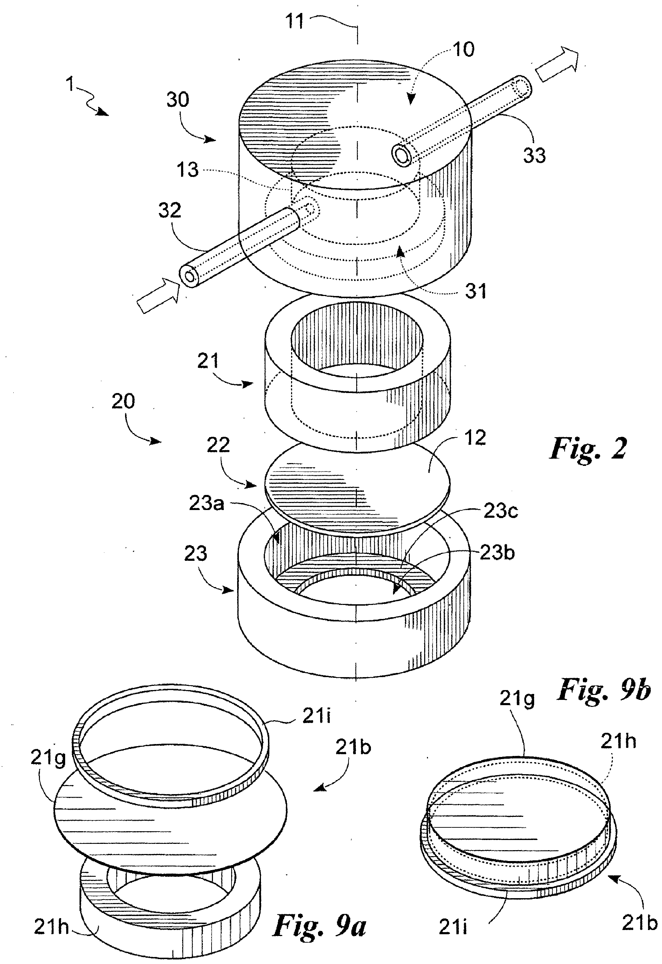

The ducts 32 and 33 have axes of extension that substantially lie on a plane that is perpendicular with respect to the supporting surface 12 and intersect the preferred axis 11. In particular, the axes of the ducts 32 and 33 are substantially parallel with one another and are reciprocally spaced at a distance of substantially less than 3 mm and, in particular, substantially equal to 1.3 mm. The first body 20, as illustrated in Figs. 1 and 2, comprises a block **21** defining the first portion of the chamber 10 and suitable to be coupled, preferably in a detachable manner, with the second body 30 to form an airtight seal; a transparent support **22,** made of glass or other plastic material suitable to permit optical access (laser stimulation) and, in particular, which is see-through, defining a supporting surface 12 substantially perpendicular with respect to the preferred axis 11; and a base **23** suitable to be coupled, preferably in a detachable manner, with the block 21 to form an airtight seal and to hold the support 22 between the block 21 and the base 23.

In particular, the coupling between the block 21 and the base 23 and between the block 21 and the second body 31 is such to permit the assembly and disassembly of said elements and, thus, define a position in which the block 21, support 22 and base 23 are reciprocally integral and a mobile position in which they are reciprocally mobile and, for instance, may be replaced. Preferably, said coupling between the base 23 and the block 21 is substantially achieved by fitting and, in detail, by means of a coupling with interference.

Note that in this description the term fitting preferably refers to a coupling with interference, i.e. a connection suitable to guarantee interference between the hole, in this case the base 23, and the element inserted in the hole, in this case the block 21, at all times, so as to be airtight and prevent the passage of fluid in correspondence with the joint between the hole and the element inserted. In detail, the term fitting refers to a coupling in which the maximum dimensions of the hole are substantially smaller than or, more in detail, substantially equal to the minimum dimensions of the object to be inserted in the hole.

The base 23 comprises a seat **23a** suitable to receive the transparent support 22 and at least partially the block 21 coupling with said block 21 by fitting; an aperture **23b** suitable to be superimposed on at least part of the transparent support 22 and having a cross-section that is substantially smaller than that of the seat 23a so as to define a supporting base **23c** for the transparent support 22 and the block 21.

The seat 23a and the aperture 23b have axes of extension that substantially coincide with the axis 11 and, preferably, the aperture 23b has a cross-section that is substantially at least equal to and, more preferably, substantially larger than the cross-section of the culture chamber 10.

The base 23 is a one-piece construction and, in particular, is made of polysiloxane, or of a silicone polymer commonly referred to as silicone, and, preferably, of polydimethylsiloxane.

Moreover, the base 23 may be appropriately provided with one or more recesses **23d** obtained in correspondence with the seat 23a and suitable to house a portion of the ducts 21e and 21f and of the second measurement ducts 42 to prevent a rotation of the first hollow element 21a with respect to the base 23.

The block 21 substantially comprises a cylindrical body having an axis that substantially coincides with the preferred axis of extension 11 and a height, calculated substantially along the preferred axis 11, that is substantially greater than that of the seat 23a so as to protrude from the base 23 and couple by fitting and, in particular, by means of coupling with interference with the second body 30 so as to define an airtight seal.

The block 21 is a one-piece construction and, in particular, is made of polysiloxane and, preferably, of polydimethylsiloxane.

Alternatively, the block 21 comprises a plurality of components suitable to be reciprocally coupled by fitting (Figs. 3, 4, 7 and 8) and, in detail, a first hollow element **21a** suitable to be inserted in the seat 23a so as to be coupled with the base 23 by fitting; a second hollow element **21b** suitable to be coupled, preferably in a detachable manner, with the first hollow element 21 and with the second body 30 so as to be arranged between them; and a divider cell support **21c** suitable to be housed in a compartment **21d** in the first hollow element 21a so as to be arranged between the elements 21a and 21b and divide the culture chamber 10 into two airtight sub-chambers **10a.**

The first hollow element 21a is appropriately a one-piece construction and, in particular, is made of polysiloxane and, preferably, of polydimethylsiloxane. The second hollow element 21b is appropriately a one-piece construction and, in particular, is made of polysiloxane and, preferably, of polydimethylsiloxane.

It is suitable to be coupled with the first hollow element 21a by fitting to obtain an airtight seal. In particular, the second hollow element 21b is suitable to be coupled with the first hollow element 21 by means of coupling with interference. Furthermore, it is suitable to be coupled with the second body 30 by fitting and, in particular by means of coupling with interference so as to define an airtight seal between them.

The divider cell support 21c is appropriately either a 3D scaffold or a membrane which is preferably porous, meaning it is provided with holes suitable to permit the passage between the sub-chambers 10a of substances having dimensions smaller than those of the holes.

It is arranged practically parallel with the supporting surface 12 at a distance from the support 22 that is substantially comprised between 4 mm and 10 mm according to the working distance of the lenses of the microscope used. Additionally, the first element 21a may comprise a feeding duct **21e** and a discharge duct **21f** suitable to permit a fluid to enter and/or leave the airtight sub-chamber 10a comprised between the transparent support 22 and the divider cell support 21c.

Said ducts 21e and 21f have a cross-section that is substantially the same as that of the respective ducts 32 and 33 or, alternatively, they have the same cross-section as one another. They also have axes of extension which lie substantially on the positioning plane of the axes of the ducts 32 and 33 and are substantially coincident or, alternatively, reciprocally staggered with respect to the ducts 32 and 33.

The second hollow element 21b is appropriately a one-piece construction and, in particular, is made of polysiloxane and, preferably, of polydimethylsiloxane. Alternatively, the second hollow element 21b may be obtained by fitting together two or more separate elements and may comprise a divisional cell support **21g** suitable to divide the culture chamber 10 into two sub-chambers 10a; an inner ring **21h** suitable to be coupled, preferably in a detachable manner, with the second body 30; and an outer ring 21i suitable to be coupled, preferably in a detachable manner, with the first hollow element 21a and to engage with the inner ring 21h so as to hold the divisional cell support 21g between the rings 21h and 21i.

The divisional cell support 21g is appropriately either a 3D scaffold or a membrane that is preferably porous, meaning it is provided with holes suitable to permit the passage between the sub-chambers 10a of substances having dimensions smaller than those of the holes.

The outer ring 21i, as illustrated in Figs. 9a and 9b, is suitable to be coupled, preferably in a detachable manner, with the inner ring 21h in correspondence with the outer surface of the inner ring 21h by fitting so as to define an airtight seal and clamp and secure the divisional cell support 21g between the rings 21h and 21i. In particular, the outer ring 21i and the inner ring 21h are suitable to be reciprocally coupled by means of a coupling with interference.

The outer ring 21i is suitable to be coupled with the first hollow element 21a by fitting to obtain an airtight seal. In particular, the outer ring 21i is suitable to be coupled with the first element 21a by means of coupling with interference.

The inner ring 21h is suitable to be coupled with the second body 30 by fitting to obtain an airtight seal. In particular, the inner ring 21h is suitable to be coupled with the second body 30 by means of coupling with interference.

Each of the rings 21h and 21i is a one-piece construction and, more precisely, is made of a rigid biocompatible plastic material and, preferably, of Delrin or Teflon.

The second body 30 is a one-piece construction and is appropriately made of polysiloxane and, preferably, of polydimethylsiloxane.

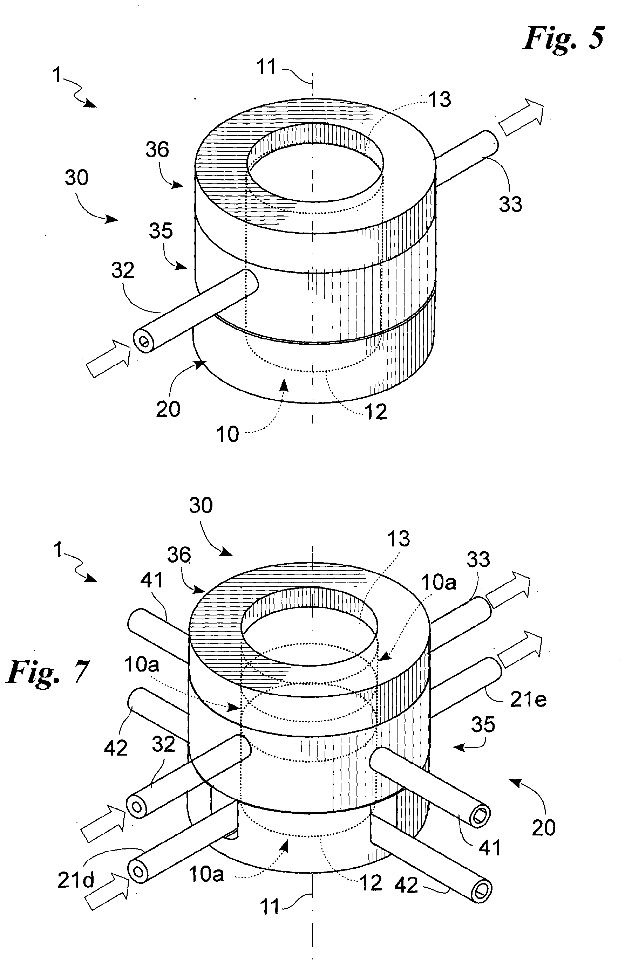

Alternatively, it comprises several components suitable to be reciprocally coupled by fitting (Figs. 5, 6, 7 and 8) so as to define a second airtight body 30. In particular, it comprises a slide **34,** made of glass or other plastic material suitable to permit optical access and, in particular, to be seen through, defining the distal surface 13 preferably substantially perpendicular with respect to the preferred axis 11; an additional block **35** defining substantially said second portion of the culture chamber 10 and a support **35a** for the slide 34 and suitable to be coupled, preferably in a detachable manner, with the block 21 and, if present, with the second element 21b; and an additional base **36** suitable to be coupled, preferably in a detachable manner, with the additional block 35 so as to hold the slide 34 between the additional block 35 and the additional base 36.

The additional block 35 is appropriately provided with the delivery duct 32 and the discharge duct 33.

The additional base 36 may be provided with an additional aperture **36a** suitable to be superimposed on at least part of the slide 22 and, preferably, with a cross-section substantially equal to the cross-section of the culture chamber 10.

The additional block 35 is a one-piece construction and is, appropriately, made of polysiloxane and, preferably, of polydimethylsiloxane.

The additional base 36 is a one-piece construction and is, appropriately, made of polysiloxane and, preferably, of polydimethylsiloxane.

The additional block 35 is suitable to be coupled with the additional base 36 by fitting to obtain an airtight seal and, in particular, by means of coupling with interference.

Moreover, the additional block 35 is suitable to be coupled, preferably in a detachable manner, with the second hollow element 21b and, thus, with the first body 20 by fitting so as to obtain an airtight seal. Preferably such coupling is obtained by means of coupling with interference.

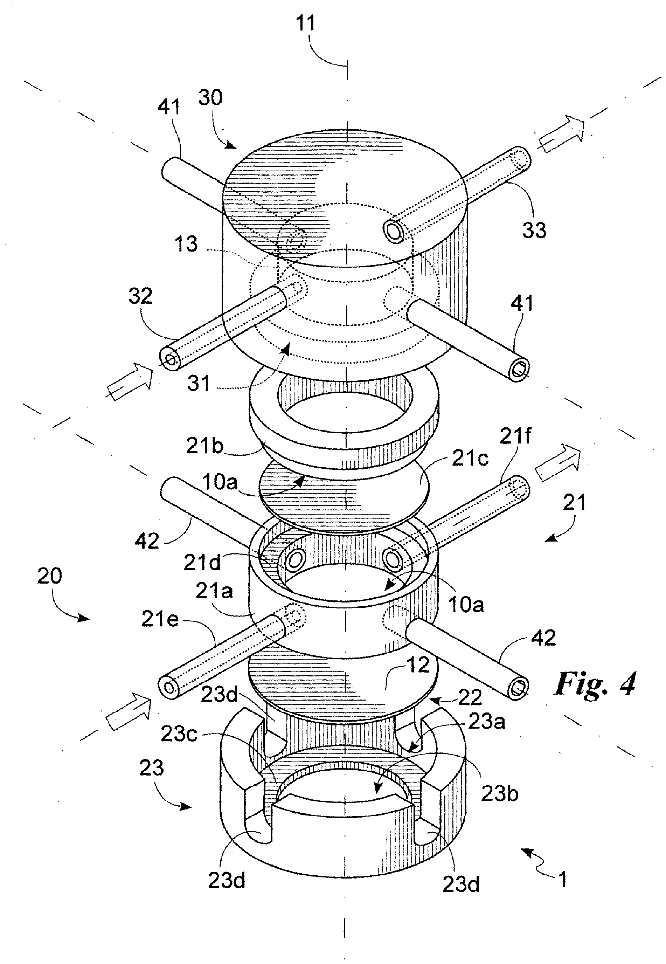

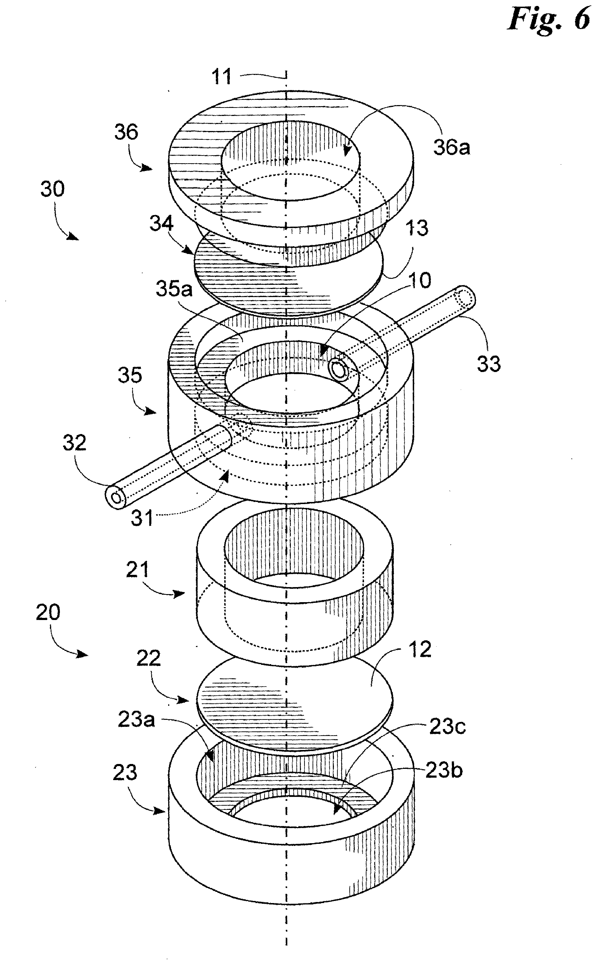

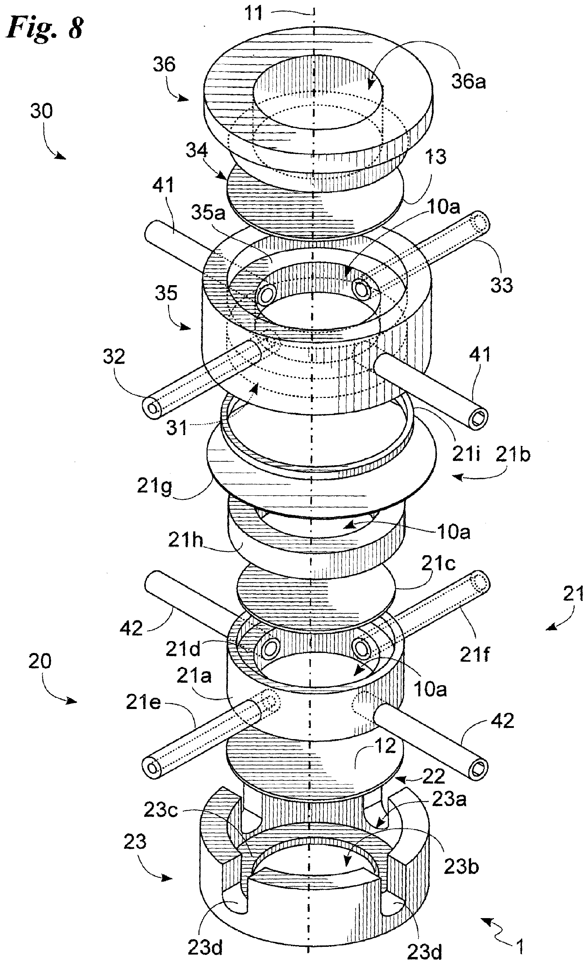

For the sake of completeness, note that the culture support 1 may have the block 21 and/or the second body 30 made as a one-piece construction or comprising a plurality of components suitable to be reciprocally coupled by fitting. Thus, according to a first embodiment (Figs. 1 and 2) the block 21 is a one-piece construction and the second body 30 is a one-piece construction; according to an alternative embodiment (Figs. 3 and 4) the block 21 comprises a plurality of components suitable to be reciprocally coupled by fitting and the second body 30 is a one-piece construction; according to further embodiment (Figs. 5 and 6) the block 21 is a one-piece construction and the second body 30 comprises a plurality of components suitable to be reciprocally coupled by fitting; and, according to a final embodiment (Figs. 7 and 8), the block 21 and the second body 30 comprise a plurality of components suitable to be reciprocally coupled by fitting.

Additionally, in the embodiments in which at least the block 21 and the second body 30 comprise a plurality of components suitable to be reciprocally coupled by fitting and, thus, the support for cell cultures 1 comprises several airtight sub-chambers 10a, the support for cell cultures 1 comprises at least one measurement duct, suitable to permit the insertion of the known measurement means into at least one of the airtight sub-chambers 10a and, thus, the measurement of the electrical impedance and/or other parameters relating to the cell construct and the fluid in the culture chamber 10.

Lastly, the support 1 may comprise (Figs. 3, 4, 7 and 8) at least a first measurement duct **41,** preferably two, suitable to allow sensing means to be inserted into and/or removed from the airtight sub-chamber 10a arranged proximal to the distal surface 13 and, in particular, between the distal surface 13 and the divisional cell support 21g; at least a second measurement duct **42,** preferably two, to allow sensing means to be inserted into and/or removed from the airtight sub-chamber 10a proximal to the supporting surface 12. Advantageously, both the first ducts 41 and the second measurement ducts 42 have substantially the same cross-section and axes of extension substantially perpendicular with respect to the axis 11 and to the axes of the ducts 32 and 33.

The assembly and functioning of a support for cell cultures described above in a structural sense are as follows.

In particular, in the embodiment of the support for cell cultures 1 illustrated in Figs. 1 and 2, assembly is performed as follows.

First, the operator inserts the transparent support 22 into the base 23 and, to be precise, into the seat 23a so that it rests on the supporting base 23c. Next the operator inserts the block 21 into the seat 23a and couples it with the base 23 by fitting and, at the same time, blocks the transparent support 22 between the resting base 23c and the block 21.

After assembling the first body 20, the operator sterilises the first body 20 and the second body 30 separately and then arranges the cell construct on the transparent support 22.

Lastly, the operator completes the assembly of the support for cell cultures 1 by coupling the second body 30 with the first body 20 by fitting and, as a consequence, creating an airtight culture chamber 10 and, more precisely, a chamber suitable to permit fluid to enter or leave exclusively via the ducts 32 and 33.

In the embodiment of the support for cultures 1 illustrated in Figs. 3 and 4, the assembly of the support for cell cultures 1 is performed as follows.

First, after sterilising the individual components of the support for cell cultures 1 separately, the operator places the support 22 in the seat 23a so that it rests against the supporting base 23c, blocks the support 22 against the base 23 by coupling the first element 21a with the base 23 by fitting so that the second measurement ducts 42 and the ducts 21e and 21f are housed in the recesses 23d and, thus, places the cell construct on the transparent support 22.

At this point, the operator completes the first body 20 by arranging the divider cell support 21c in the compartment 21d and coupling the second hollow element 21b to the first hollow element 21a by fitting so as to block the divider cell support 21c between the hollow elements 21a and 21b.

Note that this operation permits the creation of a second airtight sub-chamber 10a and, in detail, suitable to permit fluid to enter or leave exclusively through the ducts 32 and 33.

After completing the first body 20, if deemed necessary, the operator places a second cell construct on the divider cell support 21c.

Alternatively, should the operator wish to place the cell construct on the divider cell support 21c only, he first couples the base 23 to the first hollow element 21a by fitting, blocking the transparent support 22 between them, and then the two hollow elements 21a and 21b, blocking the divider cell support 21c between them. After completing the first body 20, the operator sterilises the bodies 20 and 30 separately and arranges the cell construct on the divider cell support 21c.

Lastly, the operator completes the assembly of the support for cell cultures 1 by coupling the second body 30 to the first body 20 by fitting so as to create a second airtight sub-chamber 10a and, more precisely, suitable to permit fluid to enter or leave exclusively via the ducts 32 and 33.

Once the support for cell cultures 1 is complete, the cells in the cell construct start to adhere and differentiate. At this point, without removing the transparent support 22 from the support 1, the physiological environment is mimicked by a flow generated through the ducts 32 and 33 and/or the ducts 21e and 21f.

The operator then places the support on an inverted microscope or other measurement instrument, monitors cell growth in the construct and, once the desired condition is reached, performs the necessary measurements.

In the embodiment of the support for cultures 1 illustrated in Figs. 5 and 6, the assembly of the support for cell cultures 1 is performed as follows.

First, the operator inserts the transparent support 22 into the base 23 and, to be precise, into the seat 23a so that it rests on the supporting base 23c. Next the operator inserts the block 21 into the seat 23a and couples it with the base 23 by fitting and, at the same time, blocks the transparent support 22 between the resting base 23c and the block 21.

When the first body 20 has been assembled, the operator sterilises the components of the body 30 and couples the additional block 35 with the block 21 by fitting, arranges the slide 34 on the support 35a and couples the additional base 36 with the additional block 35 by fitting, clamping the slide 34 between the additional block 35 and the additional base 36 and creating an airtight chamber 10 and, in particular, suitable to permit fluid to enter or leave exclusively through the ducts 32 and 33.

In the embodiment of the support for cultures 1 illustrated in Figs. 7 and 8, the assembly of the support for cell cultures 1 is performed as follows.

First, after sterilising the individual components of the support for cell cultures 1 separately, the operator places the support 22 in the seat 23a so that it rests against the supporting base 23c, blocks the support 22 against the base 22 by coupling the first element 21a with the base 23 by fitting so that the second measurement ducts 42 and the ducts 21e and 21f are housed in the recesses 23d and then, if desired, places a cell construct on the transparent support 22. At this point, the operator places the divider cell support 21c inside the compartment 21d, couples the inner ring 21h with the first hollow element 21a by fitting so as to block the divider cell support 21c between the inner ring 21h and the first hollow element 21a and thus create, between the support 22 and the divider cell support 21c, a first airtight sub-chamber 10a, and, in particular, suitable to permit fluid to enter or leave exclusively through the ducts 32 and 33.

Next the operator may, if he so desires, place a second cell construct on the divider cell support 21c.

At this point the operator places the divisional cell support 21g over the inner ring 21h, couples the outer ring 21i with the inner ring 21i clamping and securing the divisional cell support 21g between the rings 21h and 21i and creating a second airtight sub-chamber 10a with no ducts 32 and 33 for the delivery/discharge of fluids.

Having thus completed the first body 20, the operator couples the additional block 35 with the second element 21b by fitting, places, if he so desires, a third cell construct on the divisional cell support 21g, places the slide 34 on the support 35a and couples the additional base 36 with the additional block 35 by fitting, clamping the slide 34 between the additional block 35 and the additional base 36 and creating a third airtight sub-chamber 10a and, in particular, suitable to permit the entry or exit of fluid exclusively through the ducts 32 and 33.

The support for cell cultures 1 is now complete and ready for use.

The invention achieves some important advantages.

A first important advantage consists of the fact that the innovative support for cell cultures 1 permits the use of the transparent support 22 for analysing the cell construct without the need to remove it from the support for cultures 1 and, thus, without the risk of contaminating or altering the cell construct.

This aspect is innovatively achieved by the fact that since the block 21 and the base 23 can be reciprocally coupled by fitting, the innovative transparent support 22 can easily be inserted into the support for cultures 1 and the fact that the aperture 23b permits the inverted microscope or other instrument to perform the measurement directly on the support 22 and, thus, without the interference of other elements of the support for cell cultures 1 which would result in the degradation of said measurement.

Another advantage consists of the fact that the components of the support 1 make it possible to obtain a chamber 10 or, alternatively, a plurality of airtight sub-chambers 10a simply by fitting the various components together.

One advantage of supports with several airtight sub-chambers 10a is that, if the operator wishes to intervene on a single cell construct, leaving the other constructs unaltered, it is possible to disassemble the components of the airtight sub-chamber 10a containing the construct to be replaced and leave the other airtight sub-chambers 10a intact and, thus without interfering with the other constructs.

A further advantage, given by the superimposition of the aperture 23b and the transparent support 22, lies in the ease of monitoring the cell construct during culturing. Said aspect is further enhanced by the presence of a second body 30 comprising several components where the presence of the slide 34 offers the possibility of having another monitoring point.

An advantage is given by the presence of the divider support 21c which, by dividing the chamber 10 into airtight sub-chambers 10a, makes it possible to have substantially separate compartments and, thus, to perform complex interfacing cultures.

In particular, said aspect is particularly evident with the embodiment of the support illustrated in Figs. 3 and 4 where, for example, in the presence of two cell constructs, one on the transparent support 22 and one on the divider support 21c, it is possible to create a chamber 10 with two constructs suitable to reciprocally interact.

Furthermore, owing to the specific distance between the divider cell support 21c and the transparent support 22, the measurement instruments are even able to perform measurements on a cell construct arranged on the divider cell support 21c.

An important advantage also consists of the fact that the innovative support for cell cultures 1 is assembled by fitting, which makes it stable and easy to assemble.

A further advantage lies in the fact that with the culture chamber 10 there is no need to open a first sub-chamber 10a to access a second sub-chamber 10a, which means the first sub-chamber 10a is always kept perfectly sterilised and, thus, is suitable to guarantee an optimal culturing process.

Said aspect is further enhanced by the possibility of sterilising the various components separately and thus in the best possible way.

An additional advantage lies in the ability of the support 1 to perform analyses in real-time, that is to analyse the cell construct while it is being stimulated via the ducts 32 and 33 and/or the ducts 21e and 21f.

Moreover, the definition of an airtight sub-chamber 10a with no ducts, between the cell supports 21c and 21g, means it is possible to have a stable culture chamber that cannot be influenced from the outside and which is thus ideal for floating or non-adhesive cultures.

In the embodiment in which the support 1 has both cell supports 21c and 21g (Figs. 7 and 8), the chamber 10 is divided into three airtight sub-chambers 10a. In particular, there is an airtight sub-chamber 10a between the supporting surface 12 and the divider cell support 21c, an airtight sub-chamber 10a between the divisional cell support 21g and the distal surface 13, accessible, as is the previous airtight sub-chamber 10a, via the ducts 32, 33, 41 and 42, and an airtight sub-chamber 10a between the cell supports 21c and 21g with no ducts 32, 33 41 and 42 and the ducts 21e and 21f so as to define a stable culturing environment that cannot be influenced directly from the outside and which is thus ideal for a floating or non-adhesive culture.

Another no less important advantage is given by the specific material with which the bodies 20 and 30 are substantially made which, in addition to guaranteeing a high level of biocompatibility, are transparent to light and thus allow the cell construct to be illuminated from a surface other than the aperture 23b.

Another advantage, by no means less important, lies in the fact that the transparent support 22 and/or the slide 34 allow optical access and thus permit photostimulation of the cell construct.

Moreover, since the transparent support 22 and/or the slide 34 are see-through, the cell construct can be monitored at any time.

## Claims

1. Support for cell cultures (1) comprising a culture chamber (10) defining a supporting surface (12) for a cell construct; **characterised in that** it comprises a first body (20) defining a first portion of said culture chamber (10); and a second body (30) defining a second portion of said culture chamber complementary to said first portion; **in that** said first body (20) and said second body (30) are suitable to be reciprocally coupled so as to define said culture chamber (10); **in that** said first body (20) comprises a block (21) defining at least a portion of said culture chamber (10) suitable to be coupled with said second body (30) by fitting to obtain an airtight seal; a transparent support (22) defining said supporting surface (12); and a base (23) suitable to be coupled with said block (21) by fitting to obtain an airtight seal and holding said transparent support (22) between said block (21) and said base (23); and **in that** said base (23) comprises an aperture (23b) suitable to be superimposed on at least part of said transparent support (22) and **in that** said block (21) comprises a first hollow element (21a) suitable to be coupled with said base (23) by fitting; a second hollow element (21b) suitable to be coupled with said first hollow element (21a); and a divider cell support (21c) suitable to be arranged between said hollow elements (21a, 21b) so as to divide said culture chamber (10) into two airtight sub-chambers (10a).

2. Support for cell cultures (1) as claimed in claim 1, wherein said base (23) and said block (21) are suitable to be reciprocally coupled by means of a coupling with interference.

3. Support for cell cultures (1) as claimed in one or more of the preceding claims, wherein said second body (30) is a one-piece construction and made of polysiloxane; wherein said block (21) is a one-piece construction and made of polysiloxane; and wherein said base (23) is a one-piece construction and made of polysiloxane.

4. Support for cell cultures (1) as claimed in one or more of the preceding claims, wherein said aperture (23b) has a cross-section that is substantially at least equal to the cross-section of said culture chamber (10).

5. Support for cell cultures (1) as claimed in one or more of the preceding claims, wherein said divider cell support (21c) is substantially parallel with said supporting surface (12) and arranged at a distance from said transparent support (22) that is substantially comprised between 4 mm and 10 mm.

6. Support for cell cultures (1) as claimed in one or more of preceding claims, wherein said first hollow element (21a) is a one-piece construction and made of polysiloxane; and wherein said second hollow element (21b) is a one-piece construction and made of polysiloxane.

7. Support for cell cultures (1) as claimed in one or more of preceding claims, wherein said first hollow element (21a) comprises a feeding duct (21e) and a discharge duct (21f) suitable to permit a fluid to enter and/or leave the airtight sub-chamber (10a) comprised between said transparent support (22) and said divider cell support (21c).

8. Support for cell cultures (1) as claimed in one or more of preceding claims, wherein said second body (30) comprises a delivery duct (32) and a discharge duct (33) suitable to permit said fluid to enter and/or leave the airtight sub-chamber (10a) comprised between said second body (30) and said divider cell support (21c).

9. Support for cell cultures (1) as claimed in one or more of preceding claims, comprising at least a first measurement duct (41) suitable to permit the insertion of sensing means of said cell construct into one of said two airtight sub-chambers (10a) and at least a second measurement duct (42) suitable to permit the insertion of said sensing means into the other of said two airtight sub-chambers (10a).

10. Support for cell cultures (1) as claimed in one or more of preceding claims, wherein said second hollow element (21b) comprises a divisional cell support (21g) suitable to divide said culture chamber (10) into at least two airtight sub-chambers (10a); an inner ring (21h) suitable to be coupled with said first hollow element (21a) and an outer ring (21i) suitable to be coupled with said inner ring (21h) by fitting to hold between them said divisional cell support (21g) and to be coupled with said second body (30).

11. Support for cell cultures (1) as claimed in the preceding claim, wherein said inner ring (21h) and said outer ring (21i) are made of a rigid biocompatible plastic material.

12. Support for cell cultures (1) as claimed in one or more of the preceding claims, wherein said second body (30) comprises a slide (34), an additional block (35) substantially defining said second portion of said culture chamber (10) and suitable to engage with said block (21); and an additional base (35) suitable to be coupled with said additional block (35) by fitting to hold said slide (34) between said additional block (35) and said additional base (36).

13. Support for cell cultures (1) as claimed in the preceding claim, wherein said additional base (36) comprises an additional aperture (36a) suitable to be superimposed on at least part of said slide 22 and having a cross-section that is substantially equal to the cross-section of said culture chamber (10).

14. Support for cell cultures (1) as claimed in one or more of claims 12-13, wherein said additional block (35) and said additional base (36) are made of polysiloxane.

## Patentansprüche

1. Träger für Zellkulturen (1), umfassend eine eine Trägerfläche (12) für ein Zellkonstrukt definierende Kulturkammer (10); **dadurch gekennzeichnet, dass** er einen einen ersten Abschnitt der genannten Kulturkammer (10) definierenden ersten Körper (20) und einen einen zweiten Abschnitt der genannten Kulturkammer definierenden, zu dem genannten ersten Abschnitt komplementären zweiten Körper (30) umfasst; dass der genannte erste Körper (20) und der genannte zwei Körper (30) geeignet sind, miteinander verbunden zu werden, um die genannte Kulturkammer (10) zu definieren; dass der genannte erste Körper (20) einen mindestens einen Abschnitt der genannten Kulturkammer (10) bildenden Block (21) umfasst, der geeignet ist, mit dem genannten zweiten Körper (30) verbunden zu werden, um einen luftdichten Sitz zu erhalten; einen die genannte Trägerfläche (12) definierenden durchsichtigen Träger (22); und eine Basis (23), die geeignet ist, mit dem genannten Block (21) durch Zusammenfügen verbunden zu werden, um einen luftdichten Sitz zu erhalten und den genannten durchsichtigen Träger (22) zwischen dem genannten Block (21) und der genannten Basis (23) zu halten; und dadurch, dass die genannte Basis (23) eine Öffnung (23b) umfasst, die geeignet ist, auf mindestens einem Teil des genannten durchsichtigen Trägers (22) überlagert zu werden und darin, dass der genannten Block (21) ein erstes hohles Element (21a) umfasst, das geeignet ist, mit der genannten Basis (23) durch Zusammenfügen verbunden zu werden; ein zweites hohles Element (21b), das geeignet ist, mit dem genannten ersten hohlen Element (21a) verbunden zu werden; und einen Trenn-Zellträger (21c), der geeignet ist, zwischen den genannten hohlen Elementen (21a, 21b) angeordnet zu werden, um die genannte Kulturkammer (10) in zwei luftdichte Unterkammern (10a) zu unterteilen.

2. Träger für Zellkulturen (1) nach Anspruch 1, bei dem die genannte Basis (23) und der genannte Block (21) geeignet sind, durch eine überlagerte Verbindung miteinander verbunden zu werden.

3. Träger für Zellkulturen (1) nach einem oder mehreren der vorangegangenen Ansprüche, bei der der genannte zweite Körper (30) eine Konstruktion aus einem Stück aus Poly(organo)siloxan ist; bei dem der genannte Block (21) eine Konstruktion aus einem Stück aus Poly(organo)siloxan ist; und bei dem die genannte Basis (23) eine Konstruktion aus Poly(organo)siloxan ist.

4. Träger für Zellstrukturen (1) nach einem oder mehreren der vorangegangenen Ansprüche, bei dem die genannte Öffnung (23b) einen Querschnitt aufweist, der im Wesentlichen mindestens dem Querschnitt der genannten Kulturkammer (10) entspricht.

5. Träger für Zellkulturen (1) nach einem oder mehreren der vorangegangenen Ansprüche, bei dem der genannte Trenn-Zellträger (21c) im Wesentlichen parallel zu der genannten Trägerfläche (12) und in einem Abstand von dem genannten durchsichtigen Träger (22) angeordnet ist, der im Wesentlichen zwischen 4 mm und 10 mm liegt.

6. Träger für Zellkulturen (1) nach einem oder mehreren der vorangegangenen Ansprüche, bei dem das genannte erste hohle Element (21a) eine Konstruktion aus einem Stück aus Poly(organo)siloxan ist; und bei dem das genannte zweite hohle Element (21b) eine Konstruktion aus einem Stück aus Poly(organo)siloxan ist.

7. Träger für Zellkulturen (1) nach einem oder mehreren der vorangegangenen Ansprüche, bei dem das genannte erste hohle Element (21a) einen Einlasskanal (21e) und einen Auslasskanal (21f) umfasst, die geeignet sind, das Ein- oder Austreten einer Flüssigkeit in bzw. aus der luftdichten Unterkammer (10a) zwischen dem genannten durchsichtigen Träger (22) und dem genannten Trenn-Zellträger (21c) zu gestatten.

8. Träger für Zellkulturen (1) nach einem oder mehreren der vorangegangenen Ansprüche, bei dem der genannte zweite Körper (30) einen Ausgabekanal (32) und einen Auslasskanal (33) umfasst, die geeignet sind, das Ein- oder Austreten der genannten Flüssigkeit in bzw. aus der luftdichten Unterkammer (10a) zwischen dem genannten durchsichtigen Träger (30) und dem genannten Trenn-Zellträger (21c) zu gestatten.

9. Träger für Zellkulturen (1) nach einem oder mehreren der vorangegangenen Ansprüche, der mindestens einen ersten Messkanal (41) umfasst, der geeignet ist, das Einführen von Sensorvorrichtungen des genannten Zellkonstrukts in eine der genannten luftdichten Unterkammern (10a) zu gestatten und mindestens einen zweiten Messkanal (42), der geeignet ist, das Einführen der genannten Sensorvorrichtungen in die andere der genannten luftdichten Unterkammern (10a) zu gestatten.

10. Träger für Zellkulturen (1) nach einem oder mehreren der vorangegangenen Ansprüche, bei dem das genannte zweite hohle Element (21b) einen Trenn-Zellträger (21g) umfasst, der geeignet ist, die genannte Zellkammer (10) in mindestens zwei luftdichte Unterkammern (10a) zu trennen; einen inneren Ring (21h), der geeignet ist, mit dem genannten ersten hohlen Element (21a) verbunden zu werden und einen äußeren Ring (21i), der geeignet ist, mit dem genannten inneren Ring (21h) durch das Einfügen des genannten Trenn-Zellträgers (21g) dazwischen verbunden und mit dem genannten zweiten Körper (30) verbunden zu werden.

11. Träger für Zellkulturen (1) nach dem vorangegangenen Anspruch, bei dem der genannte innere Ring (21h) und der genannte äußere Ring (21i) aus einem steifen, biokompatiblen Kunststoff bestehen.

12. Träger für Zellkulturen (1) nach einem oder mehreren der vorangegangenen Ansprüche, bei dem der genannte zweite Körper (30) einen Schieber (34) und einen im Wesentlichen den genannten zweiten Abschnitt der genannten Kulturkammer (10) definierenden zusätzlichen Block (35) umfasst, der geeignet ist, in den genannten zusätzlichen Block (21) einzurasten, indem der genannte Schieber (34) zwischen dem genannten zusätzlichen Block (35) und der genannten zusätzliche Basis (36) fest eingefügt wird.

13. Träger für Zellkulturen (1) nach einem der vorangegangenen Ansprüche, bei dem die genannte zusätzliche Basis (36) eine zusätzliche Öffnung (36a) umfasst, die geeignet ist, auf mindestens einem Teil des genannten Schiebers (22) überlagert zu werden und einen Querschnitt aufweist, der im Wesentlichen dem Querschnitt der genannten Kulturkammer (10) entspricht.

14. Träger für Zellkulturen (1) nach einem oder mehreren der vorangegangenen Ansprüche 12-13, bei dem der genannte zusätzliche Block (35) und die genannte zusätzliche Basis (36) aus Poly(organo)siloxan bestehen.

## Revendications

1. Support pour cultures cellulaires (1) comprenant une chambre de culture (10) définissant une surface de support (12) pour une structure cellulaire ; **caractérisé en ce qu'**il comprend un premier corps (20) définissant une première partie de ladite chambre de culture (10) et un deuxième corps (30) définissant une deuxième partie de ladite chambre de culture complémentaire à ladite première partie ; **en ce que** ledit premier corps (20) et ledit deuxième corps (30) sont conçus pour être couples réciproquement de manière à définir ladite chambre de culture (10) ; **en ce que** ledit premier corps (20) comprend un bloc (21) définissant au moins une partie de ladite chambre de culture (10) conçu pour être couple audit deuxième corps (30) par emboitement pour obtenir un joint étanche à l'air ; un support transparent (22) définissant ladite surface de support (12) ; et une base (23) pouvant être couplée audit bloc (21) par emboitement pour obtenir un joint étanche à l'air et maintenir ledit support transparent (22) entre ledit bloc (21) et ladite base (23) ; et **en ce que** ladite base (23) comprend une ouverture (23b) pouvant être superposée à au moins une partie dudit support transparent (22) et **en ce que** ledit bloc (21) comprend un premier élément creux (21a) pouvant être couplé à ladite base (23) par emboitement ; un deuxième élément creux (21b) pouvant être couplé audit premier élément creux (21a) ; et un support cellulaire de division (21c) destiné à être disposé entre lesdits éléments creux (21a, 21b) de manière à diviser ladite chambre de culture (10) en deux sous-chambres étanches à l'air (10a).

2. Support pour cultures cellulaires (1) selon la revendication 1, dans lequel ladite base (23) et ledit bloc (21) sont conçus pour être couplés réciproquement au moyen d'un couplage par interférences.

3. Support pour cultures cellulaires (1) selon l'une ou plusieurs des revendications précédentes, dans lequel ledit deuxième corps (30) est une construction intégrale et composée d'un polysiloxane, et dans lequel ladite base (23) est une construction intégrale et composée d'un polysiloxane.

4. Support pour cultures cellulaires (1) selon l'une ou plusieurs des revendications précédentes, dans lequel ladite ouverture (23b) présente une coupe transversale qui est sensiblement au moins égale à la coupe transversale de ladite chambre de culture (10).

5. Support pour cultures cellulaires (1) selon l'une ou plusieurs des revendications précédentes, dans lequel ledit support cellulaire de division (21c) est sensiblement parallèle à ladite surface de support (12) et est agencé à une distance dudit support transparent (22), qui se situe sensiblement entre 4 mm et 10 mm.

6. Support pour cultures cellulaires (1) selon l'une ou plusieurs des revendications précédentes, dans lequel ledit premier élément creux (21a) est une construction intégrale et composée d'un polysiloxane ; et dans lequel ledit deuxième élément creux (21b) est une construction intégrale et composée d'un polysiloxane.

7. Support pour cultures cellulaires (1) selon l'une ou plusieurs des revendications précédentes, dans lequel ledit premier élément creux (21a) comprend une conduite d'alimentation (21e) et une conduite de décharge (21f) permettant à un fluide d'entrer et/ou de quitter la sous-chambre étanche à l'air (10a) s'étendant entre ledit support transparent (22) et ledit support cellulaire de division (21c).

8. Support pour cultures cellulaires (1) selon l'une ou plusieurs des revendications précédentes, dans lequel ledit deuxième corps (30) comprend une conduite de distribution (32) et une conduite de décharge (33) permettant audit fluide d'entrer et/ou de quitter la sous-chambre étanche à l'air (10a) s'étendant entre ledit deuxième corps (30) et ledit support cellulaire de division (21c).

9. Support pour cultures cellulaires (1) selon l'une ou plusieurs des revendications précédentes, comprenant au moins une première conduite de mesure (41) permettant l'insertion de moyens de détection de ladite structure cellulaire dans l'une desdites deux sous-chambres étanches à l'air (10a) et au moins une deuxième conduite de mesure (42) permettant l'insertion desdits moyens de détection de l'autre desdites deux sous-chambres étanches à l'air (10a).

10. Support pour cultures cellulaires (1) selon l'une ou plusieurs des revendications précédentes, dans lequel ledit deuxième élément creux (21b) comprend un support cellulaire de division (21g) permettant de diviser ladite chambre de culture (10) en au moins deux sous-chambres étanches à l'air (10a) ; une bague interne (21h) pouvant être coulée audit premier élément creux (21a) et une bague externe (21i) pouvant être couplée à ladite bague interne (21h) par emboitement pour maintenir entre elles, ledit support cellulaire de division (21g) et pouvant être couplée audit deux corps (30).

11. Support pour cultures cellulaires (1) selon la revendication précédente, dans lequel ladite bague interne (21h) et ladite bague externe (21i) sont composées d'une matière plastique rigide biocompatible.

12. Support pour cultures cellulaires (1) selon l'une ou plusieurs des revendications précédentes, dans lequel ledit deuxième corps (30) comprend une lame (34), un bloc supplémentaire (35) définissant sensiblement ladite deuxième partie de ladite chambre de culture (10) et conçu pour s'engager avec ledit bloc (21) ; et une base supplémentaire (36) conçue pour être couplée audit bloc supplémentaire (35) par emboitement pour maintenir ladite lame (34) entre ledit bloc supplémentaire (35) et ladite base supplémentaire (36)

13. Support pour cultures cellulaires (1) selon la revendication précédente, dans lequel ladite base supplémentaire (36) comprend une ouverture supplémentaire (36a) pouvant être superposée à au moins une partie de ladite lame 22 et ayant une coupe transversale qui est sensiblement égale à la coupe transversale de ladite chambre de culture (10).

14. Support pour cultures cellulaires (1) selon l'une ou plusieurs des revendications 12 et 13, dans lequel ledit bloc supplémentaire (35) et ladite base supplémentaire (36) sont composés d'un polysiloxane.
